# EUROPEAN PATENT SPECIFICATION

(11) **EP 2 954 299 B1**
(45) Date of publication and mention of the grant of the patent: **22.11.2017**
(21) Application number: 14721564.4
(22) Date of filing: 10.02.2014
(51) Int. Cl.: G01N 1/12

(54) **A SAMPLE HANDLING DEVICE**
PROBENHANDHABUNGSVORRICHTUNG
DISPOSITIF DE MANIPULATION D'ÉCHANTILLON

(30) Priority: 11.02.2013 TR 201301591
(43) Date of publication of application: 16.12.2015
(73) Proprietor: Capar Dincer, Sirma, Ankara (TR)
(72) Inventor: Capar Dincer, Sirma, Ankara (TR)
(74) Representative: Sagdiç, Turgay
(86) International application number: PCT/TR2014/000029
(87) International publication number: WO 2014/123498

(56) References cited:
- US-A- 3 955 421
- US-A- 5 219 390

## Description

### TECHNICAL FIELD

The invention is related to a sample handling device which enables to take samples (samples of living beings) from single celled invertebrate living beings which live in different depths of aqueous mediums such as lakes, ponds, barrages and sea shore zones.

### CURRENT TECHNIQUES

The depths of aqueous mediums such as lakes, ponds and barrages are not equal to each other at each location. The physical and chemical characteristics of water differ depending on changing depths and/or other environmental factors, thus each living water creature prefers to live at a depth suitable for itself. In order to be able to carry out a certain determination regarding a certain living group living in an aqueous medium, samples from all different depths possible must be taken. However, it is difficult for such a process to be carried out in lakes which have a wide water area and depth. In order to solve this problem, samples are taken from reference depths that have been pre-determined.

In order to catch the invertebrate living beings living in aqueous mediums to take samples; equipment such as, Van Dorn sampler at horizontal and vertical direction and plankton scoopers, ekman scooper, sediment sieves, sediment core samplers are used. However these equipments only give good results when used on macro vertebrate living beings, zooplanktonic and phytoplanktonic living beings. The 10 micrometer pore dimension plankton scooper gives good results when sampling gastrotricha single cell (ciliophora) examined within the protozoa phylum.

In the Chinese beneficial model application numbered CN2662643 a double sampling product with vertical dimensions in order to examine sea sources has been mentioned. With said device, examination by taking samples from small sea creatures primarily planktons are carried out.

The plankton scooper is a great tool to take samples within a water column, however it is not suitable to be used at shore zones. For this reason at shore zones, in order to take samples from living beings which attach themselves onto other living beings or non living materials such as plants, stones and shells may not be possible in many living being groups. In such areas, equipment which are fixed and which can be colonized and be attached onto the living being and which can stay put such as the living and non living materials are needed.

Document US 3955421 and especially document US 5219390 represent relevant state of the art.

### THE AIM OF THE INVENTION

The present invention aims to take samples from invertebrate living beings which live by attaching themselves on living beings or non living materials such as plants, stones and shells in aqueous medium such as lakes, ponds, barrages and shore zones. Another aim of the present invention is, to present the medium where single cell living beings live and to carry such living beings to the laboratory in order to examine them. It is an object of the present invention to provide for a sample handling device according to claim 1.

### DETAILED EXPLANATION OF THE INVENTION

The sample handling device developed in order to reach the aims of the invention has been illustrated in the attached figures, wherein said figures illustrate the following:

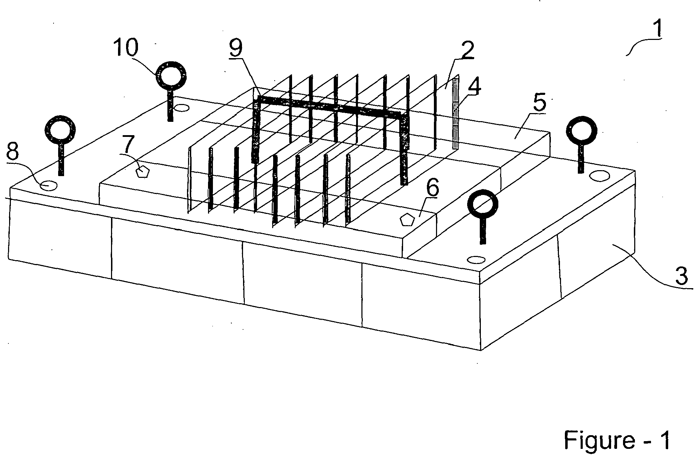

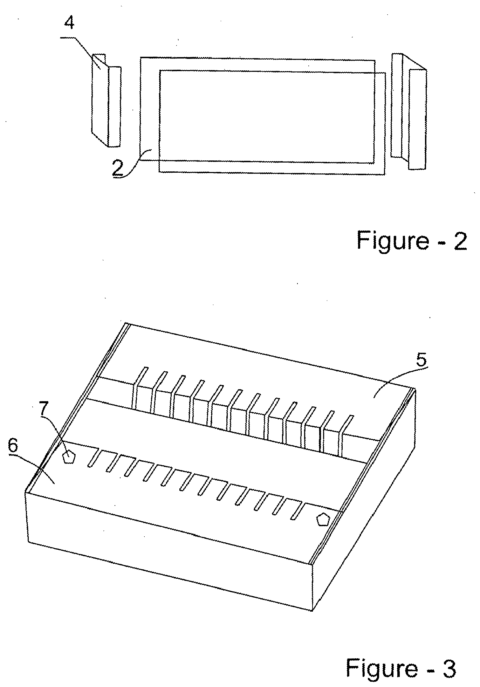

Figure 1 - General view of the sample handling device subject to the invention Figure 2 - General view of the microscope slide section of the sample handling device subject to the invention.
Figure 3 - General view of the microscope slide slot section of the sample handling device subject to the invention

The parts in the figures have each been numbered and their references have been listed below.
1. Sample handling device
2. Microscope slide
3. Weight chamber
4. Plastic frame
5. Fixed microscope slide slot
6. Mobile microscope slide slot
7. Lock
8. Fixer
9. Supporting member
10. Hook

The sample handling device (1) subject to the invention basically comprises a microscope slide (2) used for collecting samples and sampling, a fixed microscope slide (5) where the slides (2) are placed in, and a mobile slide slot (6), a plastic frame (4) used to connect two slides (2), a lock (7) used to fix the mobile slide slot (6), a supporting member (9) used to prevent the breakage of slides (2) and to ensure they stay fixed, and a hook (10).

Said sample handling device (1), is left in different depths of aqueous mediums such as lakes, ponds, barrages, and sea shore zones for a sufficient amount of time to establish a new living area. In order to represent the exact medium where single celled living beings live, by attaching themselves to solid materials, the sample handling device (1) and slides (2) are produced from transparent, light and durable materials such as PVC, or rigid glass etc.

At least 10 slides (2) are used in order to take the sufficient amount of samples from the living beings that attach themselves onto the sample handling device (1) and the number of slides (2) on the device (1) can be increased depending on the amount of samples. The slides (2) are placed in to fixed and mobile slide slots (5 and 6) in two groups by the aid of plastic frames (4) in order for one face of said slides (2) to stay clean.

The guide rails where the slides (2) have been placed in the fixed and mobile slide slot (5 and 6), need to be opened at equal width. In the case where the rail widths are different from each other the slides (2) may come out of their slots due to water flow. The slides (2) that are placed inside the guide rails within the fixed slide slot (5) can be stabilized by using a mobile slide slot (6). The mobile slide slot (6) that is used to ensure that the slides (2) do not come out of the fixed slide slot (5) are fixed to the sample handling device (1) by means of locks (7).

At least a space of 3cm is needed between the sample handling device (1) and the fixed and mobile slide slot (5 and 6) where the slides (2) are placed. Said space enables the flow of water and allows the equal distribution of living creatures that attach onto the slide (2).

In order for the sample handling device (1) to submerge into water easily, a cavity can be opened at its bottom as desired. Together with this, a weight chamber (3) is used so that the sample handling device (1) can stay fixed during the preferred amount of time, at the desired depth, without it being negatively affected from external factors such as inclination, flow etc. Additional balancing weights determined in accordance to the depth of the water can be added into the partitions within the weight chamber (3) which is mounted to the sample handling device (1) by means of fixers (8).

Ropes are used to pull back the sample handling device (1) which has been kept under water for a certain amount of time, to the surface of the water. Said ropes are tied to at least 2 preferably 4 hooks (10) that have been fixed /mounted to the corners of the bottom base of the sample handling device (1).

It is thus possible to carry the living creatures that have attached themselves on the microscopic slides (2) from their habitats, without said samples being damaged, by placing the device (1) which has been submerged into different depths of water, into a water filled container and as a result it is possible to examine said organisms while they are still living.

## Claims

1. Sample handling device (1) which enables to take samples of living beings from single celled invertebrate living beings which live in different depths of aqueous mediums such as lakes, ponds, barrages and sea shore zones, comprising:
- **a microscope slide (2)** produced from transparent, light and sustainable material such as PVC, rigid glass, etc, used for collecting samples and sampling,
- **a fixed microscope slide slot (5)** which contains the guiderails where the microscope slides (2) are placed on and which has at least a 3cm space between the sample handling device (1),
- **a mobile microscope slide slot (6)** which has at least 3cm space between the sample handling device (1) and which is used to stabilize the microscope slides (2) placed on the guide rails located in the fixed microscope slide slot (5),
- **a lock (7)** which is used to fix the mobile microscope slide slot (6) onto the sample handling device (1),
- **a supporter (9)** which is used to ensure that the slides (2) do not move and to prevent them from breaking.
- at least 2, preferably 4 **hooks (10)** that are fixed/mounted to the corner sides of the bottom base of the sample handling device (1) in order to ensure that the sample handling device (1) can be pulled back out onto the water surface,
**characterized in that** it further comprises **a plastic frame (4)** connecting two microscope slides (2) such that one face of each of the two microscope slides (2) stay clean.

2. A sample handling device (1) according to claim 1, **characterized in that** it comprises:
- **a weight chamber (3)** mounted to the sample handling device (1) which is used so that the sample handling device (1) can stay fixed during the desired period of time without being affected from external factors such as inclination, flow etc at desired water depths, and which has partitions in which additional balancing weights can be placed in accordance with the depth of the water,
- **a fixer (8)** which enables to attach the weight chamber (3) onto the sample handling device (1).

3. A sample handling device (1) according to claim 2, **characterized in that** it comprises **a cavity** opened at its bottom so that it can submerge into water easily.

4. A sample handling device (1) according to claim 1, **characterized in that** it comprises **a space** of at least 3 cm between the sample handling device (1) and the fixed and mobile slots (5 and 6), enabling the flow of water and allowing the equal distribution of living creatures.

## Patentansprüche

1. Eine Probenbehandlungsvorrichtung (1), die die Probenaufnahme von Lebewesen, nämlich von einzelligen Invertebraten ermöglicht, die in verschiedenen Tiefen der wässrigen Medien wie Seen, Teiche, Talsperren und Meeresufergebiete leben, bestehend aus:
- **einem Objektträger (2)** produziert aus transparentem, leichtem und nachhaltigem Material wie PVC, rigides Glass, usw, verwendet für Einsammlung von Proben und Probennahme,
- **einem befestigten Objektträgerschlitz (5),** der beinhaltet die Führungsschienen, wo die Objektträger (2) darauf hingelegt sind und der mindestens einen 3cm Abstand zwischen Probenbehandlungsvorrichtung (1) hat,
- **einem mobilen Objektträgerschlitz (6),** der mindestens 3cm Abstand zwischen Probenbehandlungsvorrichtung (1) hat und verwendet wird, um die Objektträger (2) auf den Führungsschienen zu stabilisieren, hingesetzt im festen Objektträgerschlitz (5),
- **einem Schlüssel (7),** der verwendet ist, um den mobilen Objektträgerschlitz (6) auf die Probenbefestigungsvorrichtung (1) zu befestigen,
- **einem Unterstützer (9),** der verwendet wird, um sicherzustellen, daß die Träger (2) sich nicht bewegen und zu verhindern, daß sie nicht gebrochen werden,
- mindestens 2, vorzugsweise 4 **Haken (10),** die fixiert/einmontiert sind zur Ecken-seiten der Untergrundlage der Probenbehandlungsvorrichtung (1), um sicherzustellen, daß die Probenbehandlungsvorrichtung (1) zurückgezogen werden kann auf die Wasseroberfläche,
**dadurch gekennzeichnet, daß** sie ferner beinhaltet **einen plastischen Rahmen (4),** der zwei Objektträgern (2) verbindet, sodaß eine Seite von jeder zwei Objektträgern (2) sauber bleibt.

2. Eine Probenbehandlungsvorrichtung (1) nach Anspruch 1, **dadurch gekennzeichnet, daß** sie bestehend aus:
- **einer Gewichtskammer (3),** einmontiert zur Probenbehandlungsvornchtung (1), die verwendet wird, sodaß die Probenbehandlungsvorrichtung (1) fest bleiben kann im Laufe der gewünschten Zeitperiode ohne von Außenfaktoren beeinflusst zu werden wie Steigung, Fluss usw. in gewünschten Wassertiefen, und die Unterteilungen besitzt, wo zusätzliche Ausgleichsgewichte hingesetzt werden können entsprechend der Wassertiefe,
- **einem Fixiermittel (8),** der ermöglicht, daß die Gewichtskammer (3) auf die Probenbehandlungsvorrichtung (1) beigefügt werden kann.

3. Eine Probenbehandlungsvorrichtung (1) nach Anspruch 2, **dadurch gekennzeichnet, daß** sie **einen Hohlraum** hat, der in seiner Grundlage geöffnet ist, sodaß sie ins Wasser leicht eingetaucht werden.

4. Eine Probenbehandlungsvorrichtung (1) nach Anspruch 1, **dadurch gekennzeichnet, daß** sie **einen Abstand** von mindestens 3 cm zwischen der Probenbehandlungsvorrichtung (1) und der befestigten und mobilen Schlitze (5 und 6) hat, die den Wasserfluss ermöglichen und eine gleichmäßige Verteilung von Lebewesen erlauben.

## Revendications

1. Dispositif de manipulation d'échantillon (1) qui permet de prélever des échantillons d'êtres vivants issus d'êtres vivants d'invertébrés uniques qui vivent dans des profondeurs différentes de milieux aqueux tels que les lacs, les étangs, les Barrages et les zones de rivage de la mer, comprenant:
- **une lame de microscope (2)** produite à partir de matériaux transparents, légers et durables tels que le PVC, le verre rigide, etc., utilisés pour la collecte d'échantillons et l'échantillonnage,
- **une fente de lame de microscope fixe (5)** qui contient les rails de guidage où les lames de microscope (2) sont placés sur et qui présente au moins un espace de 3 cm entre le dispositif de manipulation d'échantillon (1),
- **une fente de lame de microscope (6)** mobile qui présente au moins un espace de 3 cm entre le dispositif de manipulation d'échantillons (1) et qui est utilisé pour stabiliser les lames de microscope (2) placés sur les rails de guidage situés dans la fente de lame de microscope (5) fixe,
- **un verrou (7)** qui sert à fixer la fente de lame de microscope (6) mobile sur le dispositif de manipulation d'échantillon (1),
- **un supporter (9)** qui est utilisé pour s'assurer que les lames (2) ne se déplacent pas et pour éviter qu'elles ne se rompent.
- au moins 2, de préférence 4 crochets (10) qui sont fixés / montés sur les latérales d'angle de la base inférieure du dispositif de manipulation d'échantillon (1) afin de s'assurer que le dispositif de manipulation d'échantillon (1) peut être retiré sur le surface de l'eau,
**caractérisé en ce qu'**il comprend en outre un cadre en matière plastique (4) reliant les deux lames de microscope (2) de telle sorte qu'une face de chacune des deux lames de microscope (2) reste propre.

2. Dispositif de manipulation d'échantillon (1) selon la revendication 1, **caractérisé en ce qu'**il comprend:
- **une chambre de masse (3)** montée sur le dispositif de manipulation d'échantillon (1) qui est utilisée de telle sorte que le dispositif de manipulation d'échantillon (1) peut rester fixe pendant la période de temps désirée sans être affectée par des facteurs extérieurs tels que l'inclinaison, de débit etc. à souhaité profondeur de l'eau, et qui comporte des cloisons dans lesquelles masses d'équilibrage supplémentaire peut être placé en fonction de la profondeur de l'eau,
- **un fixateur (8)** qui permet d'attacher la chambre de masse (3) sur le dispositif de manipulation d'échantillon (1).

3. Dispositif de manipulation d'échantillon (1) selon la revendication 2, **caractérisé en ce qu'**il comprend une cavité ouverte à son fond de manière à pouvoir immerger facilement dans l'eau.

4. Dispositif de manipulation d'échantillon (1) selon la revendication 1, **caractérisé en ce qu'**il comprend un espace d'au moins 3 cm entre le dispositif de traitement d'échantillon (1) et les fentes fixes et mobiles (5 et 6), permettant le flux d'eau et permettant la répartition égale des êtres vivants.
